(19) Europäisches Patentamt
European Patent Office
Office européen des brevets

(11) Veröffentlichungsnummer: **0 133 881**
**A1**

(12) # EUROPÄISCHE PATENTANMELDUNG

(21) Anmeldenummer: **84106673.1**

(22) Anmeldetag: **12.06.84**

(51) Int. Cl.[4]: **C 07 C 43/13**, C 07 C 41/28, C 07 C 69/80

(30) Priorität: **08.08.83 DE 3328561**

(43) Veröffentlichungstag der Anmeldung: **13.03.85**
**Patentblatt 85/11**

(84) Benannte Vertragsstaaten: **BE DE FR GB IT NL**

(71) Anmelder: **CHEMISCHE WERKE HÜLS AG, - RSP Patente / PB 15 - Postfach 13 20, D-4370 Marl 1 (DE)**

(72) Erfinder: **Otte, Werner, Dr., Eichenstück 55, D-4270 Dorsten 11 (DE)**
Erfinder: **zur Hausen, Manfred, Dr., Höchster Strasse 1, D-4370 Marl (DE)**

(54) **Aliphatische Monoether des 2.2-Dimethylpropan-1.3-diols mit C3- bis C20-Alkoxygruppen, ausgenommen das 3-iso-Propoxy-2.2-dimethylpropan-1-ol und das 3-tert.-Butoxy-2.2-dimethylpropan-1-ol, Verfahren zu ihrer Herstellung und ihre Verwendung als Weichmacheralkohole.**

(57) Gegenstand der Erfindung sind aliphatische Monoether des 2.2-Dimethylpropan-1.3-diols mit $C_3$- bis $C_{20}$-Alkoxygruppen, ausgenommen das 3-iso-Propoxy-2.2-dimethyl-1-ol und das 3-tert.-Butoxy-2.2-dimethylpropan-1-ol.

Zu ihrer Herstellung hydriert man cyclische Acetale des 2.2-Dimethylpropan-1.3-diols mit geradkettigen oder verzweigten, gesättigten oder ungesättigten aliphatischen Aldehyden mit 3 bis 20 Kohlenstoffatomen an Kupferchromit- oder Nickelkatalysatoren bei 150 bis 350 bar und 150 bis 280 °C. Die erhaltenen Monoether des 2.2-Dimethylpropan-1.3-diols verwendet man vorzugsweise als Weichmacheralkohole.

EP 0 133 881 A1

Aliphatische Monoether des 2.2-Dimethylpropan-1.3-diols mit $C_3$- bis $C_{20}$-Alkoxygruppen, ausgenommen das 3-iso-Propoxy-2.2-dimethylpropan-1-ol und das 3-tert.-Butoxy-2.2-dimethylpropan-1-ol, Verfahren zu ihrer Herstellung und ihre Verwendung als Weichmacheralkohole

Gegenstand der Erfindung sind aliphatische Monoether des 2.2-Dimethylpropan-1.3-diols mit $C_3$- bis $C_{20}$-Alkoxygruppen, ausgenommen das 3-iso-Propoxy-2.2-dimethylpropan-1-ol und das 3-tert.-Butoxy-2.2-dimethylpropan-1-ol, Verfahren zu ihrer Herstellung und ihre Verwendung als Weichmacheralkohole zur Herstellung von Weichmachern.

Das 3-Methoxy-2.2-dimethylpropan-1-ol, das 3-Ethoxy-2.2-dimethylpropan-1-ol, das 3-iso-Propoxy-2.2-dimethylpropan-1-ol sowie das 3-tert.-Butoxy-2.2-dimethylpropan-1-ol sind bekannt. Diese Verbindungen sind ohne größere Bedeutung. Man konnte sie bisher nur nach Laborverfahren, beispielsweise durch Behandeln von 2.2-Dimethylpropan-1.3-diol mit einer aus tert.-Pentylalkohol und Natrium hergestellten Lösung und anschließende Zugabe von Methyljodid herstellen (Beilstein E IV 1, Seite $255^1$), bzw. durch Umsetzung von 2.2-Dimethylpropan-1.3-diol mit tert.-Butylalkohol in Chloroform in Gegenwart von $H_2SO_4$ (J. Org. Chem. 1968, S. 3882 und 3885). Da diese aliphatischen Monoether des 2.2-Dimethylpropan-1.3-diols, insbesondere die mit höheren Alkoxygruppen, jedoch eine Bedeutung erlangen könnten, besteht Interesse an diesen Verbindungen und einem einfachen und wirtschaftlichen Verfahren zu ihrer Herstellung.

Damit stellt sich die Aufgabe nach Schaffung eines günstigen Verfahrens, das es ermöglicht, diese neuen Monoether des 2.2-Dimethylpropan-1.3-diols mit $C_3$- bis $C_{20}$-Alkoxygruppen in einfacher und wirtschaftlicher Weise herzustellen. Diese Aufgabe wird erfindungsgemäß entsprechend den Angaben der Patentansprüche gelöst.

Überraschenderweise erhält man die Monoether des 2.2-Dimethylpropan-1.3-diols mit $C_3$- bis $C_{20}$-Alkoxygruppen durch Hydrierung der cyclischen Acetale des 2.2-Dimethylpropan-1.3-diols mit $C_3$- bis $C_{20}$ geradkettigen oder verzweigten, gesättigten oder ungesättigten aliphatischen Aldehyden, der 2-($C_2$- bis $C_{19}$-)-Alkyl- bzw. Alkenyl-5.5-dimethyl-1.3-dioxane, an Kupferchromit- oder Nickelkatalysatoren bei Drücken von 150 bis 350 bar, vorzugsweise von 200 bis 300 bar und Temperaturen von 150 bis 280 °C, vorzugsweise von 180 bis 220 °C in einfacher Weise in guter Ausbeute. Diese Reaktion war nicht zu erwarten, da nach der US-PS 4 131 739 durch Hydrierung dieser cyclischen Acetale an Ru, Rh, Pd, Os, Ir oder Pt-Katalysatoren bei Temperaturen von 200 bis 400 °C Ester entstehen.

Die cyclischen Acetale, die 2-($C_2$- bis $C_{19}$-)-Alkyl- bzw. Alkenyl-5.5-dimethyl-1.3-dioxane, erhält man in bekannter Weise durch Umsetzung von 2.2-Dimethylpropan-1.3-diol (Neopentylglykol, NPG) mit geradkettigen oder verzweigten, gesättigten oder ungesättigten aliphatischen Aldehyden mit 3 bis 20 Kohlenstoffatomen in Gegenwart von Säuren als Katalysator und Abtrennung des gebildeten Wassers. Die cyclischen Acetale haben die allgemeine Formel

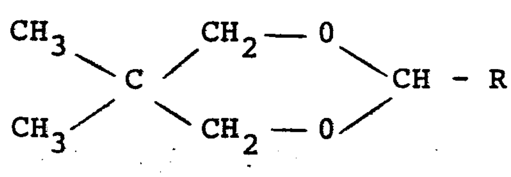

in der R geradkettige oder verzweigte, gesättigte oder ungesättigte Alkyl- bzw. Alkenylreste mit 2 bis 19 C-Atomen bedeuten.

Die Hydrierung erfolgt kontinuierlich oder diskontinuierlich bei Temperaturen von 150 bis 280 °C, bevorzugt bei

180 bis 220 °C und Drücken von 150 bis 350 bar, vorzugsweise von 200 bis 300 bar.

Als Katalysatoren sind die bekannten Kupfer- und Nickelkatalysatoren geeignet, wie Kupferchromit und Nickeloxid bzw. Kupferchromit und Nickeloxid auf inerten Trägern.
Diese Katalysatoren werden im allgemeinen mit 0,2 l Zulauf pro Liter Kontakt und Stunde belastet. Als Nebenprodukte entstehen Neopentylglykol (NPG), der gesättigte Alkohol des bei der Acetalbildung eingesetzten Aldehyds und der Diether des NPG mit der allgemeinen Formel

$$R-O-CH_2-\overset{\displaystyle CH_3}{\underset{\displaystyle CH_3}{\overset{|}{\underset{|}{C}}}}-CH_2-O-R \quad ,$$

in der R ein geradkettiger oder verzweigter Alkylrest mit 3 bis 20 Kohlenstoffatomen ist.

Den reinen Monoether erhält man durch Destillation des Hydrieraustrages.

Die als Hauptprodukt erhaltenen aliphatischen Monoether des 2.2-Dimethylpropan-1.3-diols haben die Formel

$$HO-CH_2-\overset{\displaystyle CH_3}{\underset{\displaystyle CH_3}{\overset{|}{\underset{|}{C}}}}-CH_2-O-R \quad ,$$

in der R ein geradkettiger oder verzweigter Alkylrest mit 3 bis 20 C-Atomen, ausgenommen iso-Propyl und tert.-Butyl, bedeutet, insbesondere mit 4 bis 8 C-Atomen.

Beispiele der Monoether des 2.2-Dimethylpropan-1.3-diols sind insbesondere:

3-n-Butoxy-2.2-dimethylpropan-1-ol
3-n-Hexoxy-2.2-dimethylpropan-1-ol
3-(2'-Ethylhexoxy)-2.2-dimethylpropan-1-ol

Die erfindungsgemäßen Monoether des 2.2-Dimethylpropan-1.3-diols zeichnen sich durch folgende überraschenden Eigenschaften aus: Die aus ihnen hergestellten Ester haben eine besonders geringe Flüchtigkeit und Weichmacher-Wanderung im Vergleich zu herkömmlichen Weichmacherestern mit vergleichbarem Molekulargewicht.

Daher verwendet man sie insbesondere als Weichmacheralkohole. Die mit ihnen erhaltenen Weichmacher setzt man bevorzugt zum Weichmachen von Polyvinylchlorid ein.

Die aufgeführten Beispiele sollen den Gegenstand der vorliegenden Erfindung näher erläutern, ohne ihn jedoch hierauf zu beschränken, da sich die weiteren beanspruchten aliphatischen Ether des 2.2-Dimethylpropan-1.3-diols auf entsprechende Weise herstellen lassen.

Beispiel 1

In einem Durchfluß-Hydrierreaktor mit 100 ml Volumen werden stündlich 20 ml des 2-n-Propyl-5.5-dimethyl-1.3-dioxans bei 300 bar Gesamtdruck und 160 °C an einem handelsüblichen Kupferchromitkontakt hydriert. Der Hydrieraustrag enthält im Mittel 54 % Monoether, 23 % des 1.3-Dioxans und 14 % Diether. Der Rest verteilt sich auf Butanol (3 %), NPG (5 %) und einige weitere nicht identifizierte Komponenten. Durch eine einfache Destillation im Wasserstrahlvakuum bei 93 bis 97 °C erhält man den Monoether in einer Reinheit von über 93 %, der Rest ist der Diether. Je nach Rücklaufverhältnis und Bodenzahl der verwendeten Kolonne lassen sich auch höhere Reinheiten des Monoethers erzielen.

Die beiden Komponenten des Destillats werden durch Gaschromatographie-Massenspektrometrie-Kombination sowie $^{13}C$-Kernresonanzspektrometrie identifiziert.

Physikalische Daten des 3-n-Butoxy-2.2-dimethylpropan-1-ol:

| | | | prakt. | theor. |
|---|---|---|---|---|
| $n_D^{20}$: | 1,4258 | | | |
| Dichte 20/4: | 0,8708 | C | 67,2 % | 67,5 % |
| Kp 1013 mbar: | 199 °C | H | 12,7 % | 12,5 % |
| Flammp.: | 84 °C | O | 20,3 % | 20,0 % |

Vergleichbare Ergebnisse werden erhalten, wenn man die Hydrierung bei 350 bar durchführt.

Beispiel 2

Durchführung wie Beispiel 1. Anstelle des Kupferchromitkontaktes wird ein Nickeloxidkatalysator eingesetzt. Die Hydrierung erfolgt bei 150 °C. Der Hydrieraustrag hat folgende Zusammensetzung:

54 % Monoether
23 % 1.3-Dioxan
13 % Diether
2 % Butanol
6 % NPG
2 % nicht identifizierte Verbindungen.

Beispiel 3

Ausführung wie Beispiel 2, jedoch wird die Temperatur auf 210 °C erhöht. Der Hydrieraustrag enthält im Mittel:

80 % Monoether
4 % Dioxan
6 % Diether
5 % Butanol
3 % NPG
2 % nicht identifizierte Verbindungen.

Beispiel 4

Ausführung wie Beispiel 3.
Anstelle des 2-n-Propyl-5.5-dimethyl-1.3-dioxans wird 2-(1'-Ethylpent-1-enyl)-5.5-dimethyl-1.3-dioxan bei 190 °C Hydriertemperatur eingesetzt. Der Hydrieraustrag enthält im Mittel:

| | |
|---|---|
| 66 | % Monoether |
| 1,5 | % 1.3-Dioxan |
| 16 | % Diether |
| 0,5 | % 2-Ethylhexan-1-ol |
| 6 | % NPG |
| 10 | % nicht identifizierte Verbindungen. |

Durch einfache Destillation im Wasserstrahlvakuum wird der Monoether in über 98 %iger Reinheit erhalten.
Die Identifizierung des Monoethers erfolgte wie in Beispiel 1 durch Kombination von Gaschromatographie und Massenspektrometrie sowie durch $^{13}C$-Kernresonanzspektrometrie.

Physikalische Daten des 3-(2'-Ethylhexoxy)-2.2-dimethylpropan-1-ol:

| | | | prakt. | theor. |
|---|---|---|---|---|
| $n_D^{20}$: | 1,4380 | | | |
| Dichte 20/4: | 0,8669 | C | 71,9 % | 72,2 % |
| Kp 1 013 mbar: | 254 °C | H | 13,2 % | 13,0 % |
| Flammp.: | 120 °C (PM) | O | 14,9 % | 14,8 % |

Beispiel 5

Ausführung wie in Beispiel 3.
Anstelle des 2-n-Propyl-5.5-dimethyl-1.3-dioxans wird 2-n-Pentyl-5.5-dimethyl-1.3-dioxan bei 190 °C Hydriertemperatur und 200 bar eingesetzt. Der Hydrieraustrag enthält im Mittel:

80 % Monoether
9,5 % Diether
3,5 % Acetal
3,0 % NPG
4,0 % nicht identifizierte Verbindungen.

Die Identifizierung des Monoethers erfolgte wie in Beispiel 1 durch Kombination von Gaschromatographie und Massenspektrometrie sowie durch $^{13}C$-Kernresonanzspektrometrie.

Physikalische Daten des 3-n-Hexoxy-2.2-dimethylpropan-1-ol:

| | | | prakt. | theor. |
|---|---|---|---|---|
| $n_D^{20}$: | 1,4332 | | | |
| Dichte 20/4: | 0,8688 | C | 69,8 % | 70,2 % |
| Kp 1 013 mbar: | 234 °C | H | 13,0 % | 12,8 % |
| Flammp.: | 108 °C (PM) | O | 17,1 % | 17,0 % |

Beispiel 6

Zur Prüfung der Weichmachereigenschaften der mit PSA (Phthalsäureanhydrid) veresterten erfindungsgemäßen Etheralkohole wurde stellvertretend für die erfindungsgemäßen Etheralkohole 3-n-Butoxy-2.2-dimethylpropan-1-ol (Butoxy-NPG) in bekannter Weise mit PSA verestert (S.Z.: 0,01; $n_D^{20}$: 1,4756; Dichte; 0,9654; Reinheit nach GC: $>$ 97 %). Von diesem Ester wurden PVC-Compounds hergestellt (40 Teile Ester und 60 Teile PVC) und die Weichmachereigenschaften im Vergleich zum DOP (Di-(2-Ethylhexyl)-phthalat) bestimmt.

Besonders überraschend war der deutlich geringere Gewichtsverlust und die wesentlich geringere Weichmacher-Wanderung des mit PSA veresterten Butoxy-NPG.

| | Butoxy-NPG-PSA-Ester | DOP |
|---|---|---|
| Flüchtigkeit (BS 1995/1953) | 1,3 % | 2,2 % |
| Weichmacherwanderung (DIN 53405) | 0,03 % | 3 % |

Weitere anwendungstechnische Eigenschaften, wie z. B. Shore-Härte (DIN 53505), Bruchdehnung (DIN 53455) und Lösemittelbeständigkeit sind vergleichbar.

Diese aus den erfindungsgemäßen Etheralkoholen hergestellten Weichmacher sind geeignet sowohl für die Herstellung von Weich-PVC als auch von PVC-Plastisolen (PVC-Pasten).

Patentansprüche:

1. Aliphatische Monoether des 2.2-Dimethylpropan-1.3-diols der allgemeinen Formel

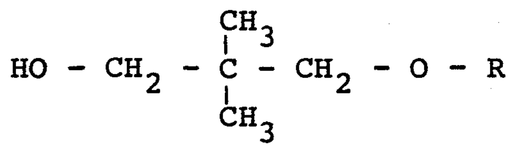 ,

in der R ein geradkettiger oder verzweigter gesättigter Alkylrest mit 3 bis 20 Kohlenstoffatomen bedeutet, ausgenommen 3-iso-Propoxy-2.2-dimethylpropan-1-ol und 3-tert.-Butoxy-2.2-dimethylpropan-1-ol.

2. Aliphatische Monoether des 2.2-Dimethylpropan-1.3-diols der allgemeinen Formel nach Anspruch 1, in der R ein n-Butyl-, ein n-Hexyl- oder ein 2-Ethylhexyl-Rest bedeutet.

3. Verfahren zur Herstellung von aliphatischen Monoethern des 2.2-Dimethylpropan-1.3-diols der allgemeinen Formel

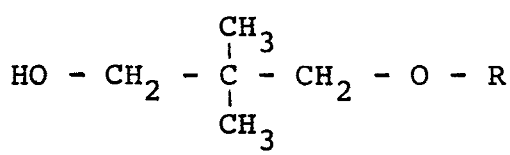 ,

in der R ein geradkettiger oder verzweigter gesättigter Alkylrest mit 3 bis 20 Kohlenstoffatomen ist,
dadurch gekennzeichnet,
daß man cyclische Acetale des 2.2-Dimethylpropan-1.3-diols mit geradkettigen oder verzweigten, gesättigten oder ungesättigten aliphatischen Aldehyden mit 3 bis 20 Kohlenstoffatomen der allgemeinen Formel

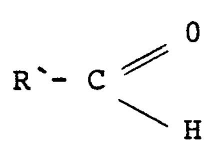 ,

in der R` ein gerader oder verzweigter Alkyl- bzw. Alkenylrest mit 2 bis 19 Kohlenstoffatomen ist, an Kupferchromit- oder Nickelkatalysatoren bei Drücken von 150 bis 350 bar und Temperaturen von 150 bis 280 °C hydriert.

4. Verwendung der Verbindungen nach Anspruch 1 und 2 als Weichmacheralkohole zur Herstellung von Weichmachern.

Europäisches Patentamt

# EUROPÄISCHER RECHERCHENBERICHT

0133881 Nummer der Anmeldung

EP 84106673.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE - B - 1 196 174 (FARBENFABRIKEN BAYER)<br>* Patentansprüche 1-3; Spalte 2, Zeile 40 - Spalte 3, Zeile 28; Beispiel 7 *<br>-- | 3,4 | C 07 C 43/13<br>C 07 C 41/28<br>C 07 C 69/80 |
| D,A | BEILSTEINS HANDBUCH DER ORGANISCHEN CHEMIE, 4. Auflage, 4. Ergänzungswerk, 1. Band, 4. Teil, 1974<br>SPRINGER VERLAG, Berlin, Heidelberg, New York<br>Seiten 2550,2551<br>* Seite 2550, Formel XI; Seite 2551, Zeilen 38-42 *<br>---- | | RECHERCHIERTE SACHGEBIETE (Int. Cl.4)<br>C 07 C 43/00<br>C 07 C 41/00<br>C 07 C 69/00<br>C 08 K |

Der vorliegende Recherchenbericht wurde für alle Patentansprüche erstellt.

| Recherchenort | Abschlußdatum der Recherche | Prüfer |
|---|---|---|
| WIEN | 19-10-1984 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82